Europäisches Patentamt

European Patent Office

Office européen des brevets

(19)

(11) Veröffentlichungsnummer: **0 042 468**
**B1**

(12) **EUROPÄISCHE PATENTSCHRIFT**

(45) Veröffentlichungstag der Patentschrift: 28.05.86

(51) Int. Cl.⁴: **A 61 B 5/08, A 61 M 15/00**

(21) Anmeldenummer: **81101411.7**

(22) Anmeldetag: **26.02.81**

(54) **Einrichtung zur Untersuchung der Atemwege auf Reizstoff-Überempfindlichkeit.**

(30) Priorität: **24.06.80 DE 3023648**

(43) Veröffentlichungstag der Anmeldung:
**30.12.81 Patentblatt 81/52**

(45) Bekanntmachung des Hinweises auf die
Patenterteilung:
**28.05.86 Patentblatt 86/22**

(84) Benannte Vertragsstaaten:
**AT BE CH DE FR GB IT LI NL SE**

(56) Entgegenhaltungen:
**BE-A- 883 449**
**DE-A-2 649 876**
**DE-A-2 809 255**
**FR-A-2 301 226**
**GB-A-2 055 046**

(73) Patentinhaber: **ERICH JÄGER GmbH & Co. KG**
**Leibnizstrasse 7**
**D-8706 Höchberg (DE)**

(72) Erfinder: **Eichler, Rüdiger, Ing. grad.**
**Wiesenstrasse 4**
**D-8705 Zellingen (DE)**

(74) Vertreter: **Heusler, Wolfgang, Dipl.-Ing. et al**
**Von Bezold, Dieter, Dr. Schütz, Peter, Dipl.-Ing.**
**Heusler, Wolfgang, Dipl.-Ing. Maria-Theresia-**
**Strasse 22 Postfach 860 260**
**D-8000 München 86 (DE)**

Courier Press, Leamington Spa, England.

### Beschreibung

Die vorliegende Erfindung betrifft eine Einrichtung zur Untersuchung der Atemwege eines Probanden auf Überempfindlichkeite gegen bestimmte Reizstoffe, mit einem an die Atemwege des Probanden anschließbaren Atemrohr, das ein Mundende aufweist, ferner mit einer Vorrichtung zum Ermitteln des Strömungswiderstandes der Atemwege, welche ein Strömungswiderstandelement sowie ein steuerbares Ventil, die dem Atemrohr in Reihe geschaltet sind, und einen an das Atemrohr angeschlossenen Druckaufnehmer enthält, und mit einem Reizstoffvernebler sowie mindestens einem weiteren Vernebler, die jeweils einen an das Atemrohr angeschlossenen Aerosol-Ausgang sowie einen Steuereingang zum Zuführen von die Vernebelung bewirkender Betriebsenergie aufweisen. Eine solche Einrichtung ist aus DE—U—79 14 971 bekannt.

Im Bronchialsystem von Patienten, die an Asthma leiden, zeigt sich auch im beschwerdefreien Intervall eine Überempfindlichkeit der Atemwege gegenüber bestimmten exogenen Reizen. Bei solchen Patienten kann durch Inhalation von Allergenen oder bestimmten aktiven Substanzen ein Bronchospasmus ausgelöst werden, der sich in einer reversiblen Erhöhung des Atemwegwiderstandes äußert. Um bei solchen Personen eine Hyposensibilisierungsbehandlung zur Heilung oder doch wenigstens zur Reduzierung des Beschwerdebildes durchführen zu können, ist es erforderlich, die das Asthma auslösenden Allergene zu bestimmen. Dies geschieht im allgemeinen mittels eines sogenannten Asthma-Provokationstestes, bei dem man den Probanden ein Aerosil atmen läßt, das ein vernebeltes Allergen enthält.

Während man früher den Probanden während einer bestimmten Zeitspanne, die eine größere Anzahl von Atemzügen umfaßte, ein Allergen-Aerosol inhalieren ließ und danach den Atemwegswiderstand maß, sodann beim Ausbleiben einer Reaktion dies mit einer höheren Allergenkonzentration wiederholte, bis ein bestimmter Atemwegswiderstand überschritten wurde, ist in jüngerer Zeit aus der deutschen Gebrauchsmusterschrift DE—U—79 14 971 eine Einrichtung bekannt geworden, die es ermöglicht, den Atemwergswiderstand während der Inhalation quasi kontinuierlich zu messen. Dies erfolgt dadurch, daß zwischen dem Vernebler und einem Mundstück, an das der Proband angeschlossen ist, ein bei der Inspiration sich öffnendes Ventil angeordnet ist und zwischen diesem Ventil und dem Mundstück eine Leitung abzweigt, die zu einer Meßvorrichtung zum Messen des Atemwegswiderstandes bei der Expiration führt. Mit einer solchen Einrichtung kann eine beginnende Atemwegsobstruktion schnell erkannt und durch sofortige Applizierung eines Gegenmittels abgefangen werden.

Aus DE—A—28 09 255 ist ferner ein Dosiersystem für die Stimulierung von Bronchialspasmen bekannt, welche einen Zerstäuber mit einem etwa birnenförmigen Gehäuse enthält, das am einen Ende ein Mundstück für den Probanden und am anderen Ende einen mit einem Strömungsfühler versehen, zur Umgebung führenden Luftkanal aufweist. Der Zerstäuber ist ferner über ein Magnetventil mit einer Druckluftquelle verbunden. Der Ausgang des Strömungsfühlers ist mit einer elekmcischen Schaltung verbunden, welche das Magnetventil eine bestimmte Zeitspanne nach Einatembeginn öffnet und für eine vorgewählte Dauer offen Hält, während der dann eine im Zerstäuber enthaltene Substanz vernebelt und von Probanden eingeatmet wird. Mit dieser bekannten Einrichtung kann lediglich eine bestimmte Substanz in Aerosolform mit vorgegebener Dosis appliziert werden, es ist mit dieser Einrichtung jedoch nicht moglich, irgendwelche Reaktionen des Probanden auf die applizierte Substanz festzustellen, geschweige denn den Widerstand der Atemwege zu messen.

Der vorliegenden Erfindung liegt die Aufgabe zugrunde, eine Einrichtung der aus DE—79 14 971 U1 bekannten Art dahingehend weiterzuentwickeln, daß die Menge des vom Probanden eingeatmeten Aerosols noch genauer bestimmbar ist und daß man einfach und schnell auf verschiedene Allergene bzw. Aerosole umschalten kann, ohne daß hierzu eine mühsame Reinigung der Einrichtung erforderlich ist und ohne daß eine nennenswerte Nachwirkung eines vorher applizierten Aerosols infolge von Rückstandbildung zu befürchten ist.

Diese Aufgabe wird bei einer Einrichtung der eingangs genannten Art erfindungsgemäß dadurch gelöst, daß die Aerosol-Ausgänge zwischen dem Mundende und dem Strömungswiderstandelement in unmittelbarer Nähe des Mundendes des Atemrohres angeordnet und mit dem Atemrohr direkt und dauernd strömungsmäßig verbunden sind, und, daß eine an sich bekannte, auf die Atemphase des Probanden ansprechende Steuervorrichtung zum Zuführen von Betriebsenergie zum Steuereingang eines vorgegebenen Verneblers während eines Teiles der Inspirationsphase mindestens eines Atemzuges vorgesehen ist.

Bei einer solchen Einrichtung wird das Aerosol nur kurzzeitig und während eines solchen Teiles der Inspirationsphase erzeugt, daß es praktisch vollständig inhaliert wird. Dadurch ist einerseits eine definierte und reproduzierbare Applikation des Aerosols gewährleistet und andererseits wird eine Verunreinigung des gerätes durch nichtinhaliertes Aerosol, das sich irgendwo absetzen könnte, vermieden.

Weiterbildungen und vorteilhafte Ausgestaltungen der erfindungsgemäßen Einrichtung sind Gegenstand von Unteransprüchen.

Im folgenden wird ein Ausführungsbeispiel der Erfindung unter Bezugnahme auf die Zeichnung näher erläutert.

Es zeigen:

Figur 1 eine schematische Darstellung einer Einrichtung gemäß einer bevorzugten Ausführungsform der Erfindung;

Figur 2 ein Diagramm zur Erläuterung der Arbeitsweise der Einrichtung gemäß Fig. 1 und

Figur 3 ein Schaltbild eines Teiles der Steuervorrichtung der Einrichtung gemäß Fig. 1.

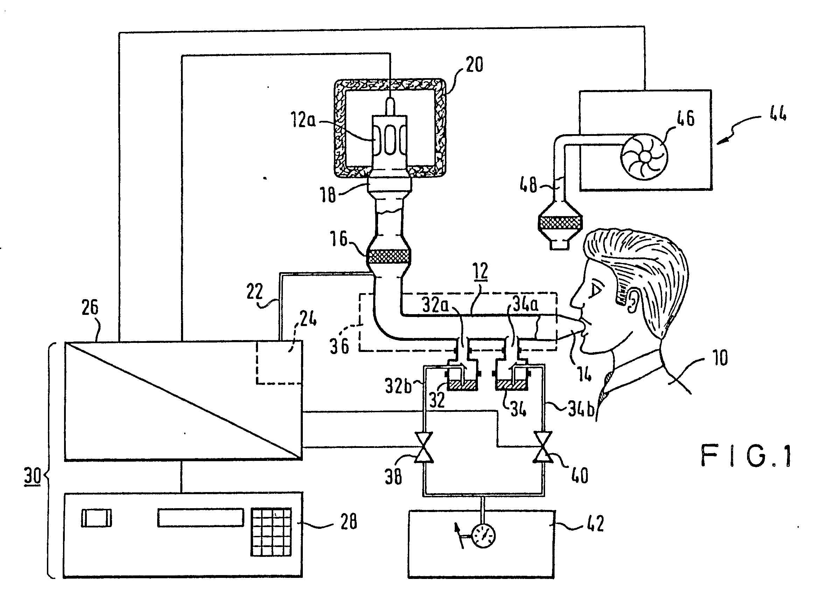

Die in Figur 1 dargestellte Einrichtung zur Untersuchung der Atemwege eines Probanden 10 auf Überempfindlichkeit gegen Reizstoffe (Allergene) enthält ein Atemrohr 12, an das der Proband 10 mittels eines Mundstückes 14 angeschlossen wird. Das Atemrohr 12 enthält einen Strömungswiderstandselement 16, z.B., in Form eines Metallnetzes, und anschließend eine schnell arbeitende, elektromagnetisch betätigte Verschlußvorrichtung. Ein sich hinter der Verschlußvorrichtung 18 befindliches Auslaßende 12a des Atemrohres ist von einem Filter 20 umgeben, um das Austreten von Aerosolen in die Umgebung zu verhindern.

An der dem Mundstück 14 zugewandten Seite des Stromungswiderstandselementes 16 ist das Atemrohr 12 über eine Druckmeßleitung 22 mit einem Druckaufnehmer 24 verbunden, der in einer Schnittstelle 26 untergebracht ist, welche zusammen mit einem Rechner 28 eine Steuervorrichtung 30 bildet, die das Gerät steuert und die bei einer Untersuchung anfallenden Daten verarbeitet.

Der sich zwischen dem Mundstück 14 und dem Stromungswiderstandselement 16 befindende Teil des Atemrohrs 12 ist außerdem mit einem Aerosol-Ausgang 32a eines Zerstäubers oder Verneblers 32 und einem Aerosolausgang mindestens eines weiteren Verneblers verbunden. Diese Vernebler 32, 34 usw. können handelsübliche, mit Druckluft betriebene Vorrichtungen sein, die an einen Block 36 ansteckbar sind, welcher in Fig. 1 nur schematisch dargestellt ist und einen Teil des Atemrohres 12 enthält.

Die Aerosol-Ausgänge der Vernebler sind direkt, d.h. ohne Zwischenschaltung eines Ventils oder dergl., mit dem Atemrohr 12 verbunden. Die Verbindung soll kurz sein und möglichst nahe beim Mundstück 14 im Atemrohr 12 münden.

Die Vernebler 32, 34 weisen ferner einen Druckluftanschluß 32b bzw. 24b auf, der einen Steuereingang zum Zuführen der die Verneblung bewirkenden Betriebsenergie darstellt. Die Druckluftanschlüsse 32b, 34b sind jeweils über ein Magnetventil 38, 40 mit einer Druckluftquelle 42 verbunden, die einen Druckregler enthält und Druckluft konstanten Druckes liefert.

Es ist ferner eine Eichvorrichtung 44 vorgesehen, welche ein Eichgebläse 46 enthält, das eine anstelle des Mundstücks 14 anschließbare Ausgangsleitung 48 aufweist, die anstelle des Mundstücks 14 an das Atemrohr 12 anschließbar ist.

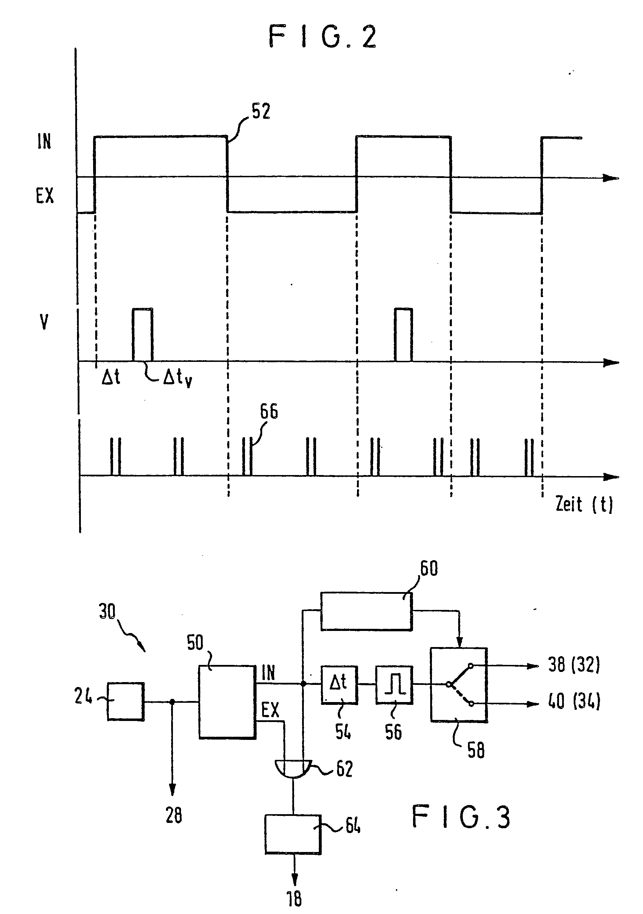

Wie Fig. 3 zeigt, ist der Druckaufnehmer 24 mit einer Schwellenwertschaltung 50 verbunden, die zwei Ausgänge IN und EX hat, an denen während der Inspirationsphase bzw. der Expirationsphase ein Ausgangssignal auftritt, wie die Kurve 52 in Fig. 2 zeigt. Das Signal IN wird dem Eingang eines Verzögerungsgliedes 54 zugeführt, das eine vorteilhalterweise einstellbare Zeitspanne $\Delta t$ nach Beginn der Inspirationsphase einen Impuls an einen Monovibrator 56 liefert, der einen Ausgangsimpuls, dessen Dauer vorzugsweise ebenfalls einstellbar ist, an den Eingang eines Umschalters 58 liefert. Der Umschalter 58 hat einen ersten Ausgang, der zum Ventil 38 des Verneblers 32 führt und einen zweiten Ausgang, der zum Ventil 40 für den Vernebler 34 führt. Wenn der Vernebler 38 reines Lösungsmittel und der Vernebler 34 Lösungsmittel mit Reizstoff enthält, so ist der Ausgang des Monovibrators 56 im Ruhezustand des Schalters mit dem Ventil 38 des Verneblers 32 verbunden.

Der Umschalter 58 wird durch einen Zähler 60 gesteuert, dessen Eingang ebenfalls das Signal IN zugeführt ist. Durch die Einstellung des Zählers 60 läßt sich das Verhältnis der Anzahl der Atemzüge, während derer der Reizstoffvernebler in Betrieb gesetzt wird, zur Anzahl der Atemzüge, in denen der Lösungsmittelvernebler in Betrieb gesetzt wird, nach Wunsch einstellen, z.B. im Bereich von 1:100 bis 10:1.

Die Ausgänge IN und EX sind außerdem über ein ODER-Glied 62 mit einem Impulsgenerator 64 verbunden, der während jeder Inspirations- und Expirationsphase einen oder mehrere Impulse 66 (Fig. 2) zum Schließen der Verschlußvorrichtung 18 und damit zur Messung des Atemwegwiderstandes nach dem Verschlußdruckprinzip liefert.

Der Ausgang des Druckaufnehmers 24 ist außerdem mit einem Datenspeicher des Rechners 28 verbunden, um die übliche Atemluftströmungskurve und dgl. zu erfassen.

Das beschriebene Gerät arbeitet folgendermaßen: Am Anfang und während eines späteren Teiles jeder Inspirationsphase und Expirationsphase werden ein oder mehrere Impulse 66 erzeugt, die den Verschluß 10 schließen, so daß sich im Atemrohr ein Druck entsprechend dem Alveolardruck einstellt, aus dem zusammen mit der vor der Betätigung des Verschlusses 18 herrschenden Strömungsgeschwindigkeit in bekannter Weise der Atemwegswiderstand errechnet werden kann. Bei der zu Beginn jeder Untersuchung durchgeführten Bestimmung des Atemwegswiderstandes des Probanden sind alle Vernebler außer Betrieb oder es wird nur der Vernebler eingeschaltet, der ausschließlich Lösungsmittel enthält.

Zu Beginn des eigentlichen Asthma - Provokationstestes wird der Zähler 60 so eingestellt, daß er nur nach Ablauf der vorgewählten Inspirationsphaseneinen Umschaltimpuls an den Umschalter 58 liefert. Zu Beginn jeder Inspirationsphase erhält das Verzögerungsglied 54 einen Eingangsimpuls und nach Ablauf der Verzögerungszeit $\Delta t$ wird der Monovibrator 56 ausgelöst und liefert einen "Verneblungs"-Impuls V. Durch diesen Impuls wird der durch den Umschalter 58 angeschaltete Vernebler 32 oder 34 während einer kurzen Zeitspanne innerhalb jeder Inspirationsphase in Betrieb gesetzt. Die Dauer dieser Zeitspanne Δt ist vorzugsweise wesentlich kürzer als eine normale Inspirationsphase. Die Verzögerungszeit Δt wird so gewählt, daß der Impuls V bei einem Atemzug mit normaler Inspirationsdauer etwa in die erste Hälfte der Inspirationsphase fällt.

Diese Art der Steuerung der Vernebler hat folgende wesentliche Vorteile.

1) wird das ganze Aerosol, das während der kurzen Zeitspanne Δt$_V$ erzeugt wird, von der Inspirationsluft mitgerissen und auch ganz eingeatmet. Die Dosierung ist dadurch sehr exakt und hängt nicht von der Dauer der Inspiration ab;

2) setzt sich praktisch kein Aerosol im Atemrohr 12 ab, so daß die Verunreinigung des Atemrohrs und damit auch eine unkontrollierte Rückatmung von Aerosol weitgehend vermieden werden;

3) wird praktisch kein Aerosol ausgeatmet, da das ganze Aerosol bis in die Alveoli gelangt und Pendelluft in den Bronchien und dem Mundraum wegen der frühzeitigen Injektion des Aerosols in dei Inspirationsluft kein Aerosol enthält. Die ausgeatmete Luft enthält daher kaum unabsorbiertes Aerosol.

Es soll in diesem Zusammenhang bemerkt werden, daß die geregelte Druckluftquelle 42 durch Wahl des Betriebsdruckes einen Betrieb der Vernebler 32, 34 in einen optimalen Betriebsbereich zuläßt, bei dem Nebeltröpfchen solcher Größe entstehen, daß sie optimal aufgenommen werden.

Bei der erfindungsgemäßen Einrichtung kann mit einer festen Verdünnung des Reizstoffes gearbeitet werde, da die Dosierung auch über das Verhältnis der Aerosol-Atemzüge mit und ohne Reizstoffsubstanz erfolgt.

Ein weiterer vorteil der vorliegenden Einrichtung besteht darin, daß der Totraum gering ist und daß praktisch nur der Teil des Atemrohres zwischen dem Anschluß der Vernebler und dem Mündstuck mit Aerosol in Berührung kommen kann. Hierdurch wird die Reinigung sehr erleichtert. Ein Substanzwechsel ist einfach und schnell durchzuführen, da nur der Reizstoffvernebler ausgetauscht zu werden braucht. Gewünschtenfalls kann zusätzlich noch ein Vernebler (nicht dargestellt) vorgesehen sein, der ein Gegenmittel zum Abfangen der provozierten Asthmareaktion enthält.

Die Steurung des durch den Umschalter 58 ausgewählten Verneblers kann auch vorteilhalterweise in Abhängigkeit von der Strömungs geschwindigkeit der Inspirations erfolgen. In diesem Falle wird dann der die Dauer der Reizstoff-Vernebelung bestimmende Monovibrator 56 durch das Ausgangssignal des Druckaufnehmers 24 über ein Schwellenwertglied, dessen Schwellenwert vorzugsweise einstellbar ist, gesteuert.

Die Funktion des Zählers 60 kann vom Rechner 28 erfüllt werden. Der Umschalter 58 kann mehr als zwei Schaltpositionen enthalten, wenn mehr als zwei Vernebler vorgesehen sind.

**Patentansprüche**

1. Einrichtung zur Untersuchung der Atemwege eines Probanden auf Überempfindlichkeit gegen bestimmte Reizstoffe, mit einem an die Atemwege des Probanden anschließbaren Atemrohr (12), das ein Mundende (14) aufweist, ferner mit einer Vorrichtung zum Ermitteln des Strömungswiderstandes der Atemwege, welche ein Strömungswiderstandselement (16) sowie ein steuerbares Ventil (18), die dem Atemrohr (12) in Reihe geschaltet sind, und einen an das Atemrohr angeschlossenen Druckaufnehmer (24) enthält, und mit einem Reizstoffvernebler (34) sowie mindestens einem weiteren Vernebler (32), die jeweils einen an das Atemrohr (12) angeschlossenen Aerosol-Ausgang (32a, 34a) sowie einen Steuereingang (32b, 34b) zum Zuführen von die Vernebelung bewirkender Betriebsenergie aufweisen, dadurch gekennzeichnet, daß die Aerosol-Ausgänge (32a bzw. 34a) zwischen dem Mundende (14) und dem Strömungswiderstandselement (16) in unmittelbarer Nähe des Mundendes (14) des Atemrohres (12) angeordnet und mit dem Atemrohr (12) direkt und dauernd strömungsmäßig verbunden sind; und daß eine an sich bekannte, auf die Atemphase des Probanden ansprechende Steuervorrichtung (30) zum Zuführen von Betriebsenergie zum Steuereingang (32b, 34b) eines vorgegebenen Verneblers (32, 34) während eines Teiles der Inspirationsphase (IN) mindestens eines Atemzuges vorgesehen ist.

2. Einrichtung nach Anspruch 1, dadurch gekennzeichnet, daß die Steuervorrichtung eine Schaltung (54, 56) enthält, die eine Betätigung der Vernebler (32, 34) während der ersten zwei Drittel, insbesondere während der ersten Hälfte der Inspirationsphase eines normalen Atemzuges bewirkt.

3. Einrichtung nach Anspruch 2, gekennzeichnet durch eine Anordnung (54) zur Einstellung der Zeitspanne (Δt) zwischen dem Beginn einer Inspirationsphase (IN) und dem Einschalten der Vernebler (32 bzw. 34).

4. Einrichtung nach Anspruch 2, dadurch gekennzeichnet, daß die Vernebler (32, 34) durch eine auf die Strömungsgeschwindigkeit der Inspirationsluft ansprechende Anordnung einschaltbar sind.

5. Einrichtung nach Anspruch 2, 3 oder 4, dadurch gekennzeichnet, daß die Dauer der Betätigung der Verneblers (34) höchstens 500 Millisekunden beträgt.

6. Einrichtung nach einem der Ansprüche 1 bis 5, gekennzeichnet Verneblung von Lösungsmittel durch eine Schaltvorrichtung (58, 60), durch die Reizstoffvernebler (34) und der weitere Vernebler (32), der zur Verneblung von Lösungsmittel dient, während der Inspirationsphase der verschiedenen Atemzüge wahlweise einschaltbar sind.

7. Einrichtung nach einem der Ansprüche 1 bis 6, gekennzeichnet durch eine Anordnung (60) zur Einstellung des Verhältnisses der Anzahl der Atemzüge, während derer der Reizstoffvernebler (34) eingeschaltet wird, zur Anzahl der Atemzüge, während derer der Reizstoffvernebler nicht in Betrieb gesetzt wird.

8. Einrichtung nach einem der vorhergehenden Ansprüche, dadurch gekennzeichnet, daß die Zeitdauer ($\Delta T_V$) der Einschaltung des jeweiligen Verneblers (32, 34) einstellbar ist.

9. Einrichtung nach einem der vorhergehenden Ansprüche mit durch Druckluft gespeisten Verneblern, dadurch gekennzeichnet, daß jeder Vernebler (32, 34) über ein durch die Steuervorrichtung (30) steuerbares Ventil (38 bzw. 40) mit einer druckgeregelten Druckluftquelle (42) verbunden ist.

10. Einrichtung nach einem der vorhergehenden Ansprüche, dadurch gekennzeichnet, daß die Vernebler (32, 34) an das Atemrohr (12) ansteckbar sind.

**Revendications**

1. Dispositif d'évaluation d'allergies des voies respiratoires d'un patient provoquées par des substances irritantes déterminées, comprenant un tube respiratoire (12) pouvant être raccordé aux voies respiratoires du patient, qui présente une extrémité (14) destinée à la bouche, et en outre un dispositif pour déterminer la résistance au passage du courant dans les voies respiratoires, qui contient une élément de résistance au courant (16) ainsi qu'une soupape réglable (18) montés en série avec le tube respiratoire (12), et un capteur de pression (24) raccordé au tube respiratoire, et comprenant un vaporisateur de substances irritantes (34) ainsi qu'au moins un autre vaporisateur (32), qui présentent chacun une sortie d'aérosol (32a, 34a) raccordée au tube respiratoire (12) ainsi qu'une entrée de commande (32b, 34b) destinée à l'amenée de l'énergie d'entraînement provoquant la vaporisation, caractérisé par le fait que les sorties d'aérosol (32a, 34a), sont disposées entre l'extrémité (14) destinée à la bouche et l'élément de résistance au courant (16), à proximité immédiate de l'extrémité réservée à la bouche (14) du tube respiratoire (12), et sont reliées directement et de façon permanente au tube respiratoire (12) de façon à établir le courant; et en ce qu'il est prévu un dispositif de commande (30) connu en soi, répondant à la phase respiratoire du patient, en vue d'amener l'énergie d'entraînement à l'entrée de commande (32b, 34b) d'un vaporisateur prédéterminé (32, 34) pendant une partie de la phase d'inspiration (IN) d'au moins une respiration.

2. Dispositif selon la revendication 1, caractérisé par le fait que le dispositif de commande comprend un circuit (54, 56) provoquant le fonctionnement des vaporisateurs (32, 34) pendant les premiers deux tiers, et en particulier pendant la première moitié de la phase d'inspiration d'une respiration normale.

3. Dispositif selon la revendication 2, caractérisé par un agencement (54) pour régler la durée (t) entre le commencement d'une phase d'inspiration (IN) et la mise en circuit des vaporisateurs (32 ou 34).

4. Dispositif selon la revendication 2, caractérisé par le fait que les vaporisateurs (32, 34) peuvent être mis en circuit par l'intermédiaire d'un agencement sensible à la vitesse du courant de l'air d'inspiration.

5. Dispositif selon la revendication 3 ou 4, caractérisé par le fait que la durée de fonctionnement des vaporisateurs (32, 34) est d'au maximum 500 millisecondes.

6. Dispositif selon l'une des revendications 1 à 5, caractérisé par un dispositif de commutation (58, 60) au moyen duquel on peut brancher au choix le vaporisateur de substances irritantes (34) et l'autre vaporisateur (32) qui sert à la vaporisation d'un solvant pendant la phase d'inspiration des diverses respirations.

7. Dispositif selon l'une des revendications 1 à 6, caractérisé par un agencement (60) pour régler le rapport entre le nombre de respirations pendant lesquelles le vaporisateur de substances irritantes (34) est branché et le nombre de respirations pendant lesquelles le vaporisateur de substances irritantes n'est pas en service.

8. Dispositif selon l'une des revendications précédentes, caractérisé par le fait que la durée ($T_V$) du branchement du vaporisateur respectif (32, 34) est réglable.

9. Dispositif selon l'une des revendications précédentes, caractérisé par le fait que chaque vaporisateur (32, 34) est relié à une source d'air comprimé (42) régulée en pression, par l'intermédiaire d'un soupape (38 ou 40) qui peut être commandée par le dispositif de commande (30).

10. Dispositif selon l'une des revendications précédentes, caractérisé par le fait que les vaporisateurs (32, 34) peuvent être enfichés dans le tube respiratoire (12).

**Claims**

1. A device for the evaluation of hypersensibility of the respiratory ducts of an experimentee to certain stimulants including a respiratory tube (12) which can be connected to the respiratory ducts of the experimentee, which tube has a mouthpiece (14), and a device for determining the resistance to flow of the respiratory ducts which includes a flow resistance element (16) and a controllable valve (18) which are connected in series with the respiratory tube (12), and a pressure absorber (24) connected to the respiratory tube, and including a stimulant atomizer (34) and at least one further atomizer (32), each atomizer having an aerosol outlet (32a, 34a) connected to the respiratory tube (12) and a control inlet (32b, 34b), for supplying operational energy effecting the atomization, characterised in that the aerosol outlets (32a and 34a) are arranged between the mouthpiece (14) and the flow resistance element (16) in the direct vicinity of the mouthpiece (14) of the respiratory tube (12) and in direct constant flow connection with the respiratory tube (12); and that a control device (30) is provided which is known per se and responsive to the respiratory phase of the experimentee for supplying operational energy to the control inlet (32b, 34b) of a predetermined atomizer (32, 34) during one part of the inhalation phase (IN) of at least one breath.

2. A device according to claim 1, characterized in that the control device includes a circuit (54, 56) which effects an actuation of the atomizers (32, 34) during the first two thirds, especially during the first half of the inhalation phase of a normal breath.

3. A device according to claim 2, characterised by an arrangement (54) for adjusting the time span (Δt) between the beginning of an inhalation phase (IN) and the switching of the atomizers (32 and 34).

4. A device according to claim 2, characterised in that the atomizers (32, 34) can be switched on by an arrangement responding to the velocity of flow of the inhaled air.

5. A device according to claim 2, 3 or 4, characterised in that the duration of the operation of the atomizers (34) is 500 milliseconds at the most.

6. A device according to one of claims 1 to 5, characterised by a switching device (58, 60) by which the stimulant atomizer (34) and the further atomizers (32) which serve to atomize solvents can be selectively switched on during the inhalation phase of the different breaths.

7. A device according to one of claims 1 to 6, characterised by an arrangement (60) for adjusting the relationship of the number of breaths during which the stimulant atomizer (34) is switched on to the number of breaths during which the stimulant atomizer is not put into operation.

8. A device according to one of the preceding claims, characterised in that the duration (ΔT_v) of the operation of each atomizer (32, 34) is adjustable.

9. A device according to one of the preceding claims, with atomizers fed with compressed air, characterised in that each atomizer (32, 34) is connected to a pressure-regulated compressed air source (42) via a valve (38 and 40) which can be controlled by the control device (30).

10. A device according to one of the preceding claims, characterised in that the atomizers (32, 34) can be plug connected to the respiratory tube (12).

F I G . 1

0 042 468

F I G. 2

F I G. 3

2